# EUROPEAN PATENT APPLICATION

(11) **EP 4 768 002 A1**
(43) Date of publication of application: **01.07.2026**
(21) Application number: 24223837.6
(22) Date of filing: 31.12.2024
(51) Int. Cl.: A61F 13/00, A61B 5/103, A61B 5/00

(54) **MULTI-LAYERED DRESSING FOR MONITORING SKIN CHANGES BY USE OF A COMPUTER-IMPLEMENTED METHOD**

(71) Applicant: Dermatoo, 4053 Chaudfontaine (BE)
(72) Inventor: DELARBRE, Julien, 4053 Embourg (BE); DELARBRE, François, 4219 Ambresin (BE); DOYEN, Sébastien, 1200 Woluwe-Saint-Lambert (BE)
(74) Representative: Calysta NV

(57) **Abstract**

A multi-layered dressing configured for monitoring skin changes over time of a skin of a subject by use of a computer-implemented method, the dressing comprising a first layer configured to be in contact with the skin during use of the dressing, and a second layer arranged on top of the first layer, wherein the second layer comprises a calibration reference detectable by an imaging sensor operatively connectable with a computing system for capturing data for use in the computer-implemented method.

## Description

### Technical Field

The present invention relates to a dressing configured for monitoring skin changes of a subject by use of a computer-implemented method, in particular a multi-layered dressing for covering a wound.

### Prior art

Dressings are applied on a skin to provide protection from the external environment, in several medical and cosmetic applications.

For example, dressings can be applied on wounds, burns, canker sores or ulcers to protect against infections and/or mechanical trauma and promote healing. Dressings may also ensure maintenance of adequate moisture levels, absorption of liquids (e.g. dressings for heavily exuding wounds), transdermal delivery of active compounds (e.g., of antimicrobial compounds, analgesic compounds, growth factors), and/or provide compression (e.g. to reduce bleeding or swelling).

Dressings can be applied to the skin in various manners. For example, non-adhesive dressings can be fixed on the skin using tape and/or bandages; adhesive dressings incorporate a self-adhesive layer (e.g. made of silicone) that can adhere to the skin without the need for external fixation; island dressings comprise a central non-adhesive layer surrounded by an adhesive border.

Dressings may comprise one or more layers. When multiple layers are present, these can be made of different dressing materials. Examples of dressing materials are gauze, cotton, rayon, polyester, hydrocolloids, hydrogels, polyurethane, silicone, alginates. These materials can be in the form of textile materials (e.g. woven or nonwoven fabrics) or non-textile materials. Some materials, such as alginates, may exist in both textile and non-textile form.

A major drawback of dressings is their inability to facilitate visual observation of the underlying skin tissues without removal of the dressing. Frequent dressing changes for observation can cause trauma to the skin, increase the risk of contamination, and disrupt the local environment maintained by the dressing, potentially delaying the healing process.

Transparent dressings facilitate visual observation of the underlying skin without removing the dressing.

However, these dressings are unsuitable for wounds with heavy exudate, and their application is limited to specific wound types.

Furthermore, observation through a transparent dressing is influenced by external factors such as lighting conditions, variability arising from differences in observers' experience, and device or technique used to perform observation. These sources of inconsistency complicate the effective tracking of the skin healing process and can lead to subjective or inaccurate assessments.

Therefore, existing solutions are inadequate for ensuring accurate monitoring of skin conditions requiring the application of dressings.

Accurate skin monitoring is highly desirable to draw meaningful conclusions and generate quantitative data, which is crucial for skin conditions of wounds, either chronic or not. For example, chronic wounds present significant challenges due to their prolonged healing times. Inadequately treated chronic wounds are thus at a higher risk of infection, which can lead to severe negative consequences on mobility and quality of life, particularly in the elderly and individuals with underlying conditions such as diabetes.

The preceding discussion of the prior art is intended to facilitate an understanding of the present invention only. The discussion is not an acknowledgement or admission that any of the material referred to is or was part of the common general knowledge at the priority date of the application.

### Brief summary of the invention

It is an object of the invention to overcome the disadvantages of the prior art, or at least provide an alternative to the prior art. It is in particular an object of the invention to provide a dressing that is configured to be in contact with a portion of skin of a subject and that enables accurate and consistent skin monitoring using a computer-implemented method.

According to the invention, this object is solved by a multi-layered dressing according to the first claim configured for monitoring skin changes over time of a skin of a subject by use of a computer-implemented method, the dressing comprising a first layer configured to be in contact with the skin during use of the dressing, and a second layer arranged on top of the first layer, wherein the second layer comprises a calibration reference configured to be detectable by an imaging sensor when operatively connectable with a computing system for capturing data for use in the computer-implemented method.

In the context of the invention, the term " **dressing"** refers to a skin care device designed to be applied to a portion of skin of a subject for medical or cosmetic purposes and that comprises one or more layers of dressing material.

For example, a dressing may be applied on a skin alteration, to provide protection from external factors and/or to maintain a specific local environment for the skin alteration, such as an environment that promotes healing or that preserves desired moisture levels. Examples of skin alterations are wounds e.g., wounds from injuries or surgical wounds, pressure ulcers, leg or foot ulcers, diabetic ulcers, abrasions, lacerations, burns, bedsores.

In the context of the invention, the term "**skin**" refers to a surface that forms the outermost layer of the body, and on which a dressing can be applied.

This surface can be a cutaneous surface (e.g. the epidermis) or a mucosal surface (e.g. the mucosa lining the oral cavity). When the cutaneous surface and/or the mucosal surface is breached (e.g. due to an injury) the outermost layer of the body may comprise underlying tissues that become exposed (e.g., dermis, submucosa, or subcutaneous tissues).

A "**multi-layered dressing**" is a dressing comprising two or more layers, with each layer generally serving a specific function. Multi-layered dressings comprise at least a contact layer designed to interface directly with the skin (e.g., with a wound or a burn) and an outer layer designed to interface with the external environment.

The contact layer may be an adherent layer (for example, for applications where it is desirable to provide a secure dressing), or a non-adherent layer (for example, for applications where it is desirable to prevent trauma during dressing changes).

In cases where the contact layer is an adherent layer, the contact layer may comprise a moisture-sensitive adhesive. For example, the contact layer may comprise a material that becomes sticky upon exposure to moisture. These embodiments are particularly advantageous for application of the dressing in moist environments e.g. in the oral cavity.

The outer layer may be designed to protect against external environments (e.g. the outer layer may be waterproof for applications where accidental exposure to moisture needs to be minimized), to regulate gas exchange (e.g. the outer layer may comprise perforations), and/or to improve adhesion of the dressing to the skin (e.g., the outer layer is adhesive and extends beyond the dimensions of the contact layer, thus forming an adhesive border or some adhesive flaps).

In the context of the invention, the "**first layer**" and the "**second layer**" respectively refer to the contact layer and the outer layer.

Optionally, the multi-layered dressing further comprises one or more intermediate layers between the first layer and the second layer.

The first layer, the second layer and (when present) the one or more intermediate layers may be made of different dressing materials, such as gauze, cotton, starch, cellulose, rayon, polyester, hydrocolloids, hydrogels, polyurethane (e.g. polyurethane-based foam or polyurethane-based film), silicone (e.g. silicone-based adhesive), alginates, chitosan, or collagen, or a combination thereof.

These materials can be in the form of textile materials (e.g. woven or nonwoven fabrics) or non-textile materials.

The material (or combination of material) and/or the textile or non-textile form can be selected based on target properties of that layer, such as moisture retention, moisture absorption, transparency, and/or protection from external factors (e.g. against infections, fluids, and/or mechanical trauma).

Each of the first layer, the second layer or (when present) the one or more intermediate layers may be selected from a group of an absorptive layer (e.g. made of polyurethane foam, and/or nonwoven fibers), a cooling layer (e.g., comprising hydrogels that can provide a cooling effect), an antimicrobial layer (e.g., comprising an antimicrobial agent, and/or silver particles), a drug release layer (e.g., comprising analgesic compounds, or growth factors), and/or a fluid transfer layer (e.g. having a mesh structure designed to facilitate the movement of wound exudate away from the wound, typically towards an absorptive layer).

According to embodiments, the first layer comprises the second layer, or vice-versa.

In this case, the first layer and the second layer are combined into a layer that performs the functions of both the first layer and the second layer. The combined layer thus comprises a first side designed to interface with the skin, and a second side opposite to the first side, the second side being designed to interface with the external environment. When reference is made to the second layer in continuation of this disclosure, it should thus be understood that it may comprise one side of the first layer when the first layer comprises the second layer.

The second layer comprises a calibration reference. The "**calibration reference**" is an object comprising standard, or at least observable or detectable, visual information (like color, brightness, dimensions, texture and the like) which can be placed within the field of view of an image-capturing devices and used to correct captured images and compensate for variations in capture conditions across different captured images.

For example, the calibration reference may be detectable by a 2D imaging sensor configured to capture 2D images, e.g. a visible-light image sensor or a 2D imaging sensor operating in another spectral range, or by a 3D imaging sensor configured to capture 3D images, e.g. a structured light sensor configured to detect distortions of a known projected light pattern that is reflected from the calibration reference, a Time-of-Flight (ToF) sensor, a LiDAR (configured to detect a time for emitted light to travel to and from the calibration reference).

The imaging sensor may be housed within the computing system for capturing data for use in the computer-implemented method (e.g., in a smartphone, a laptop, or a tablet) or in a different, e.g. remote, system. For example, the computing system may have functions distributed over a cloud infrastructure and be available to users as a cloud-based service.

The imaging sensor is operatively connectable with a computing system such as a smartphone, a laptop, a tablet, for capturing data for use in a computer-implemented method for monitoring skin changes over time of the skin of the subject.

The multi-layered dressing with the calibration reference being present at the outer layer thereof is thus custom made for the purpose of using a computer-implemented method for monitoring skin changes by capturing and/or detecting the calibration reference with an imaging sensor. This imaging sensor is operatively connectable with a computing system such that data captured and/or detected by the sensor can be processed by the computer system through a computer-implemented method. Furthermore, the imaging sensor can be integrated into the computer-system, for example being a camera of a smartphone.

The computer-implemented method is for monitoring skin changes over time on the basis of captured images. For a better understanding of the novel and innovative aspect of the multi-layer dressing, the computer-implemented method is further discussed. The applicant retains the option to amend the device claims related to the multi-layered dressing by incorporating references to the computer-implemented method, thereby emphasizing its novelty and inventiveness.

For example, the method may comprise the steps of:
- capturing a guidance reference image comprising a portion of the skin of the subject;
- capturing a first image of a set of monitoring images comprising the portion of the skin of the subject and further comprising the multi-layered dressing according to any one of the embodiments herein described, the multi-layered dressing being adhered to the skin;
- calculating a similarity metric between the guidance reference image and the first image of the set.

In this case, during the capturing of subsequent images of the set of monitoring images, the guidance reference image may be displayed as an overlay based on the similarity metric thereby providing guidance to a user during capturing.

By displaying the guidance reference image "**as an overlay**" based on the similarity metric, it is meant that the overlaid guidance reference image is superposed on, or blended with, the subsequent image, while allowing portions of the subsequent image to remain at least partially visible. Thus, during capturing, information is provided to the user about how closely the current capture conditions match the intended capture conditions.

For example, the overlay comprises applying a transparency coefficient to the guidance reference image and/or generating a mask of the guidance reference image.

The guidance reference image may be displayed as a semi-transparent layer and/or a mask superposed on, or blended with, a subsequent monitoring image, e.g. in a live image preview of the subsequent monitoring image.

This advantageously enables, when capturing an image of the multi-layered dressing (the first image), the display of skin features that are obscured from view to an external observer (e.g., the user and/or the subject) due to being covered by the multi-layered dressing, but which were visible in a previously captured guidance reference image. For example, it is possible to display the rims of a wound, a burn, ulcer, a sore, a blister or a similar skin alteration as an overlay on the dressing, thereby providing a visualization of the edges of the skin alteration as they could be seen by an external observer in the absence of the dressing.

In embodiments, the method further comprises a step of calibrating the guidance reference image based on the calibration reference.

In embodiments, one or more steps of the computer-implemented method (e.g. capturing, calibrating, processing, and/or analyzing data) can be performed in real-time while the multi-layered dressing remains (at least partially) in contact with the skin.

In embodiments, at least one portion of the second layer is detachably attached to the first layer, the detachable portion of the second layer comprising at least a part of the calibration reference.

For example, detachment may allow the removal of a central section of the calibration reference while a peripheral section of the calibration reference remains adhered to the first layer.

When part of the calibration reference is detachable, the user (e.g. a caregiver) can detach the detachable part of the calibration reference from the rest of the dressing, before application of the dressing. This detachable part can be placed on the skin near a skin alteration (e.g. a wound) that is intended to receive the dressing.

In this case, the guidance reference image may be an image of a portion of the skin comprising the skin alteration uncovered by the dressing and further comprising a nearby portion of the skin on which the detachable part of the calibration reference is adhered.

The dressing comprising the remaining part of the calibration reference may be applied to the skin alteration in a second step.

In this case, the first image of the set of monitoring images may be an image of a portion of the skin comprising the skin alteration covered by the dressing and further comprising the nearby skin on which the detachable part of the calibration reference is adhered.

The calibration reference may comprise a geometric reference for spatial calibration and/or a color reference for color calibration.

A "**geometric reference**" is a calibration reference having known geometrical information that is used to determine geometric transformation data (e.g., a rotation or distortion matrix) between captured images, e.g. to compensate distortions caused by differences in the orientation of the imaging device relative to the subject's skin.

A "**color reference**" is a calibration reference having known color information that is used to determine color transformation data (e.g., a color correction matrix comprising components like hue values, chrominance values, luminance values, or a combination thereof) between captured images, e.g. to compensate for different environmental lighting conditions.

When a "**color correction matrix**" is used, this matrix may enable adjustments for white balance, gamut mapping between color spaces (e.g., RGB to sRGB and/or to CIE LAB), non-linear transformations like gamma correction, spectral sensitivity alignment, noise and chromatic aberration reduction, uniformity correction for sensor inconsistencies, and/or dynamic adaptation to varying lighting conditions, typically calibrated using reference targets or environmental sampling.

For example, the geometric reference may comprise, according to an embodiment, a one-dimensional marker (e.g., a barcode, a ruler), a two-dimensional marker (e.g., an Aruco marker, a two-dimensional barcode, a QR code), or a board of two-dimensional markers (e.g., an Aruco board).

For example, the geometric reference may comprise a three-dimensional marker (e.g. a known three-dimensional shape such as a cube or a hemisphere).

For example, the color reference may comprise one or more of the group of a red marker, a yellow marker, a brown marker, a black marker, a grey marker.

In embodiments, the calibration reference comprises at least one color reference and at least one geometric reference. For example, the calibration reference may comprise a plurality of color references and geometric references arranged in a grid pattern.

The calibration reference may comprise at least one environment-reactive marker.

The **environment-reactive marker** is configured to change in visual appearance in response to a change of an environmental parameter (e.g., pH, temperature, moisture, oxygen levels). The change in visual appearance may be for instance a color shift, a color darkening, a change in texture, a swelling, a change in transparency.

Advantageously, the integration of the calibration reference into the dressing ensures that the calibration reference is positioned in a consistent location in images captured of the skin portion where the multi-layered dressing is applied. This eliminates or reduces the need for frequent manual placements of a separate calibrator, simplifying the imaging process and reducing potential errors in spatial alignment and analysis.

This also ensures accurate calibration across sequential images and facilitates accurate monitoring of skin changes over time by imaging. For example, each time the dressing is to be replaced during the skin monitoring process, it is possible to capture an image of the bare skin (e.g., of a wound) prior to applying the multi-layered dressing, followed by capturing a second image immediately after the multi-layered dressing is applied, thereby ensuring that each captured image of the uncovered skin is paired with a corresponding image comprising the calibration reference.

Advantageously, the geometric features (e.g. patterns, shapes), spectral features (e.g. color, reflectivity at specific wavelengths) and/or environmentally sensitive properties of the calibration reference ensure that the calibration reference is detectable by a wide range of image-capturing devices, from consumer-grade imaging systems (e.g., smartphone cameras) to advanced diagnostic equipment.

In embodiments where the calibration reference comprises a three-dimensional marker, the multi-layered dressing provides the advantage of enabling more comprehensive spatial calibration. Therefore, it is possible to obtain reliable depth and volume data from the calibrated images.

Moreover, in these embodiments, an accurate three-dimensional reconstruction of the monitored skin can be obtained (e.g., a wound topography), which is particularly advantageous for providing information to support clinical decision-making, especially in the context of remote health monitoring. The three-dimensional reconstruction can be obtained from 3D images based on measurements from a 3D imaging sensor (e.g. ToF, LiDAR), or from a 2D imaging sensor (e.g. in case of 3D image reconstruction from a plurality of 2D images using a photogrammetry-based technique).

In embodiments where the calibration reference comprises an environment-reactive marker, the multi-layered dressing provides the advantage of enabling real-time monitoring of environmental changes (e.g. due to bacterial metabolism or external environmental conditions), such as changes in pH, oxygen levels, temperature, or moisture. This allows for prompt intervention, avoiding the need to wait until the next scheduled change of the dressing.

In embodiments where the multi-layered dressing comprises a transparent first layer, with the second layer leaving part of the transparent layer exposed (e.g. because the second layer only partially covers the transparent layer, or because the second layer is in part detachable), calibration of images of the skin is possible while allowing observation through the transparent layer.

In embodiments where the second layer is partly detachable, and this detachable portion comprises part of the calibration reference, the detachable portion can be adhered to nearby skin while the rest of the second layer and calibration reference remain over the monitored area.

These embodiments advantageously allow to provide a dual calibration using the part of the calibration reference remaining on the dressing and the part of the calibration reference adhered to nearby skin.

Dual calibration improves calibration accuracy for imaging, since the calibration reference part on the nearby skin can be used as an external reference (or control) unaffected by skin deformations or other biological processes occurring under the dressing. It is therefore possible to obtain, from the calibrated images, reliable data for clinical evaluation.

The improved calibration accuracy also minimizes the need for manual input, thereby facilitating automated image analysis processes such as wound segmentation and/or tissue classification. Therefore, it is possible to obtain reliable data for clinical evaluation from the calibrated images, even during at-home monitoring by untrained users.

The partly detachable second layer is particularly advantageous in embodiments where the calibration reference comprises environment-reactive markers, as this allows a comparative analysis of the conditions beneath the dressing (e.g., at the site of the skin alteration) with those of the nearby skin. For example, differences in the visual appearance changes of the environment-reactive markers on the non-detachable portion and the detached portion adhered to nearby skin may indicate localized phenomena, such as bacterial activity, inflammation, or specific wound healing processes. Conversely, similar visual appearance changes in both portions may be indicative of a broader or external environmental factor.

In embodiments, the first layer comprises a moisture-absorbing material. In embodiments, at least a portion of the first layer is transparent.

For example, a transparent first layer can be made of a polyurethane film. In embodiments, the first layer is a drug-release layer.

The drug-release layer allows the release of a drug. Drug release can be passive (e.g., by diffusion) or active (e.g., triggered by conditions such as temperature, pH, or moisture).

In embodiments, the first layer is adhesive. The entire first layer may be adhesive or only parts of it (for example, the first layer has an adhesive border, or adhesive flaps).

Having an adhesive first layer has the advantage that additional bandage or skin tape is not needed. This also reduces the risk that the first layer moves or becomes detached from the skin while removing the second layer, in embodiments where the second layer is (at least partly) detachable.

In embodiments, the second layer entirely covers the first layer. For example, the second layer may extend beyond the perimeter of the first layer. The parts of the second layer extending beyond the perimeter of the first layer may be adhesive.

In embodiments, the second layer partially covers the first layer. For example, the second layer may cover a central portion of the first layer.

In embodiments, at least one portion of the second layer is detachably attached to the first layer, the detachable portion of the second layer comprising at least a part of the calibration reference.

When one or more intermediate layers are present, the detachable portion of the second layer may be detachably attached to the outermost intermediate layer.

For example, the detachable portion may be peeled away from the rest of the dressing (e.g. from the first layer or from the outermost intermediate layer).

For example, the detachable portion of the second layer may comprise a central portion of the calibration reference.

In this case, when the detachable portion is removed, the remaining part of the calibration reference defines a boundary around an opening through which the first layer (or an intermediate layer, if present) is exposed. If the exposed layer is transparent, this configuration enables direct observation of the skin alteration covered by the multi-layered dressing.

Various options exist to provide an (at least partially) detachable second layer. For example, the first layer and the second layer may be heat-sealed according to a non-continuous pattern, such that certain areas are left unsealed or less securely bonded together. This allows to provide weak points that facilitate detachment. It is also possible to define detachable regions by providing pre-cut lines in the second layer, e.g. by mechanical cutting or laser perforation.

In embodiments, the calibration reference is detectable by an imaging sensor operating in the visible spectrum.

In embodiments, the calibration reference comprises a geometric reference.

In embodiments, the geometric reference comprises a one-dimensional marker.

For example, the one-dimensional marker may comprise a barcode, or a ruler.

In embodiments, the geometric reference comprises a two-dimensional marker.

For example, the two-dimensional marker may comprise one or more of the group of an Aruco marker, a two-dimensional barcode, a QR code.

In embodiments, the geometric reference comprises a three-dimensional marker.

For example, the three-dimensional marker may comprise a known three-dimensional shape such as a cube, a hemisphere, a prism, a (truncated) pyramid, a (truncated) cone, and the like.

In embodiments, the calibration reference comprises a color reference.

For example, the color reference may comprise one or more of the group of a red marker, a yellow marker, a black marker, a brown marker, a grey marker.

These color markers are particularly advantageous to monitor different skin tissue types, such as (healthy) epithelial tissue, necrotic tissue, granulation tissue, fibrous tissue, slough, eschar, as well as fluid such as blood or serous exudate.

This further allows monitoring changes in skin tissue over time, such as a transition from necrotic tissue to granulation tissue and epithelial tissue, and/or to track the presence of fluids like blood or serous exudate.

In embodiments, the calibration reference comprises at least one color reference and at least one geometric reference.

This allows simultaneous measurement of geometric parameters and color parameters of the portion of the skin, thereby ensuring a more comprehensive monitoring of the skin. For example, it is possible to monitor whether a color shift is accompanied by a geometric change.

In embodiments, the calibration reference comprises at least one color reference and at least one geometric reference arranged in a grid pattern.

In embodiments, the geometric reference (e.g. one or more one-dimensional markers or two-dimensional markers of the calibration reference) is colored. In this case, the colored geometric reference can be used for both color calibration and geometric calibration.

In embodiments, the calibration reference comprises at least one environment-reactive marker configured to change in visual appearance in response to a change of an environmental parameter.

For example, the change in visual appearance may comprise one or more of a color shift, a change in texture, a swelling, a change in transparency.

For example, the change in visual appearance may be triggered when the environmental parameter is above a predefined threshold value.

For example, the environmental parameter may comprise one or more of a group of: pH, temperature, moisture, oxygen levels.

The change in visual appearance may occur in response to variations in an environmental parameter through a predefined relationship. For example, a gradual increase in the environmental parameter may trigger a corresponding gradual change in the visual appearance of the environment-reactive marker, such as a darkening of color. The visual change may occur continuously or incrementally as the parameter deviates further from a baseline value.

The environment-reactive marker may be configured to change in visual appearance when the environmental parameter exceeds a predefined threshold value. In this case, minor changes in the parameter below this threshold do not trigger a visual appearance change. For example, the change in visual appearance may be a visual cue, such as a symbol (e.g., an exclamation mark) or a text, that becomes visible when the threshold is exceeded. For example, the visual cue may indicate that the dressing requires replacement or intervention.

The environment-reactive marker may be configured to undergo more than one change of its visual appearance in response to a change of the environmental parameter. For example, the environment-reactive marker may undergo a gradual darkening in color while the environmental parameter changes, with a further distinct visual cue becoming visible when said environmental parameter exceeds a threshold.

The calibration reference may comprise an identification marker.

The identification marker may contain identification data relating to the dressing and or to the calibration reference.

For example, the identification data can comprise one or more of a serial number, a batch number, a product identifier, an expiration date, a production date, regulatory compliance data (e.g., a certification code and/or a certification date),a manufacturer, dimensions, type of calibration reference (e.g. "grid patterned reference"), data relating to the color markers (e.g. common color names or official designations according to a color standard), data relating to the geometric markers (e.g., shape, scale, coordinates), version, revision.

The identification marker may be a text (e.g., a serial number) or a machine-readable element such as QR code (e.g. redirecting to a document containing identification data).

In embodiments, the multi-layered dressing is selected from a group of a wound dressing, an anti-microbial dressing, a compression dressing, a burn dressing, a drug delivery dressing, a foam dressing.

According to a second aspect, the invention further relates to a method for manufacturing a multi-layered dressing according to any of the preceding claims.

In embodiments, the method comprising the steps of printing the calibration reference on the second layer, and applying the second layer on top of the first layer.

For example, the second layer may be applied by ultrasonic welding, heat-sealing, lamination, adhesive bonding (e.g. by applying a medical-grade adhesive), pressure-sensitive adhesion, laser welding.

The techniques for applying the second layer may be selected based on the materials of the first layer and/or the second layer (for example, laser welding or ultrasonic welding may be selected for layers made of thermoplastic materials), based on an intended application of the dressing (e.g. techniques ensuring strong bonding can be selected for dressings designed to adhere on the skin for extended periods and/or on skin areas subject to frequent movement), and/or based on compatibility of the technique with sterile manufacturing conditions.

In embodiments where the first layer comprises the second layer (or vice-versa), the method may comprise a step of printing the calibration reference on the first layer (or the second layer).

For example, the calibration reference may be printed on the second side of the first layer (or of the second layer).

Other embodiments according to the present invention are mentioned in the appended claims and the subsequent description of an embodiment of the invention.

### Brief description of the Drawings

Exemplary embodiments of the invention are described using the figures. It is to be understood that these figures merely serve as examples of how the invention can be implemented and are in no way intended to be construed as limiting for the scope of the invention and the claims. Like features are indicated by like reference numerals along the figures. In the figures:
**Fig. 1** is a schematic view of a multi-layered dressing comprising a first layer and a second layer, wherein the second layer comprises a calibration reference;
**Fig. 2** is a schematic view of a multi-layered dressing comprising a first layer and a second layer, wherein the second layer comprises a detachable portion comprising at least part of a calibration reference;
**Fig. 3** is a schematic view of a multi-layered dressing comprising a first layer and a second layer, wherein the second layer comprises a detachable portion comprising a calibration reference;
**Fig. 4** is a schematic view of a multi-layered dressing comprising a first layer and a second layer, wherein a portion of the second layer comprising at least part of a calibration reference is detached;
**Fig. 5A, 5B and 5C** are schematic views of three steps of a method for capturing images of a skin portion of a subject using the multi-layered dressing.

In the drawings, the same reference numbers have been allocated to the same or analogue element.

### Detailed description of embodiments of the invention

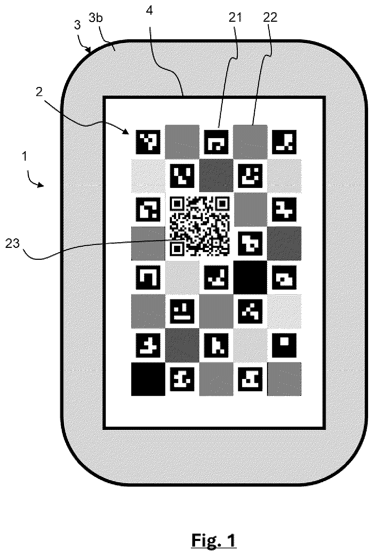

**Fig. 1** is a schematic top view of a multi-layered dressing 1 comprising a first layer 3 and a second layer 4, the second layer 4 comprising a calibration reference 2.

In this example, the second layer 4 partially covers the first layer 3. In particular, the second layer 4 is centrally aligned on the first layer 3, covering a central portion of the first layer 3 while leaving peripheral regions 3b of the first layer 3 exposed. These peripheral regions 3b may be designed to facilitate removal (e.g. by peeling) of the multi-layered dressing 1. The peripheral regions 3b (or the entire first layer 3) may be adhesive to ensure secure attachment to the skin. However, in different embodiments, the second layer 4 may have the same surface area as the first layer 3 or may extend beyond the first layer 3.

In this example, the first layer 3 comprises a first side designed to interface with the skin during use, and a second side opposite to the first side and designed to interface with the second layer. However, the first side and second side of the first layer 3 may be arranged on the same side. For example, the second layer 4 may cover the first side of the first layer 3 and is removed prior use, exposing the first side of the first layer, which is then adhered to the skin.

The calibration reference 2 comprises a geometric reference 21 and/or a color reference 22, respectively comprising a plurality of two-dimensional markers and color markers. In this example, the two-dimensional markers and color markers are arranged in a grid pattern, forming a board of alternated markers. However, the calibration reference 2 may comprise a different arrangement of the two-dimensional markers and color markers.

The two-dimensional markers can be Aruco markers, as shown in **fig. 1****,** However, different two-dimensional markers (e.g. two-dimensional barcodes) or one-dimensional markers (e.g., rulers) can also be used.

The geometric reference 21 (in this example, the Aruco markers) can be used to adjust geometric parameters of images captured of the skin portion on which the multi-layered dressing 1 is applied, e.g. to compensate for distortion, rotation, or orientation.

This allows to improve accuracy of geometric measurements done on sequentially captured images of the skin portion for monitoring skin changes over time.

Geometric measurements may comprise for instance a length, a width, a depth, a surface, or a combination thereof (e.g. a surface/perimeter ratio).

In the example of **fig. 1****,** the calibration reference 2 comprises a color reference 22 that, in this case, comprises five color markers that are repeated and alternated with the two-dimensional markers, forming a board. For example, the color markers may comprise one or more of a red marker, a yellow marker, a brown marker, a black marker, and a grey marker. However, different color markers can be selected e.g. based on the skin process that is monitored over time.

The color reference 22 can be used to adjust color parameters of images captured of the skin portion on which the multi-layered dressing 1 is applied, e.g. to compensate for variations in lighting conditions.

This allows to improve accuracy of color measurements done on sequentially captured images of the skin portion for monitoring skin changes over time.

Color measurements may comprise for instance a number of pixels classified in a predefined tissue class (e.g. fibrous tissue, necrotic tissue, blood, exudate or epithelial tissue) based on their color.

The geometric and/or color measurements may be repeated on each image of the set of monitoring images to monitor changes of the skin over time.

In the example of **fig. 1****,** the calibration reference 2 also comprises a QR code 23. The QR code 23 may store relevant metadata (e.g. batch number, expiration date, and/or manufacturer of the multi-layered dressing 1) or provide access to instructions, patient data, or software for analyzing captured images.

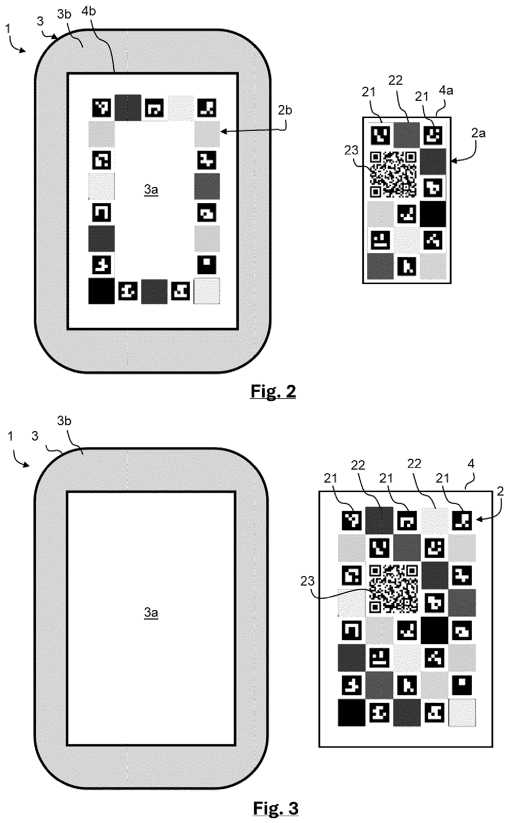

**Fig. 2** is a schematic top view of a multi-layered dressing 1 comprising a first layer 3 and a partially detachable second layer 4. The left part of **fig. 2** shows the rest of the multi-layered dressing 1 after detachment (e.g., by peeling) of the detachable portion 4a of the second layer 4. On the right side of the figure, the detached portion 4a is shown.

The detachable portion 4a of the second layer 4 comprises a first part 2a of the calibration reference 2. In this example, the detachable portion 4a of the second layer 4 comprises a central portion 2a of the calibration reference 2.

The portion 4b of the second layer 4 remaining on the dressing 1 after detachment comprises a second part 2b of the calibration reference 2. In this example, the second part 2b comprises a color reference 22 comprising a plurality of color markers, and a geometrical reference comprising a plurality of two-dimensional markers. The first part 2a of the calibration reference 2 also comprises color markers and two-dimensional markers, and it further comprises a QR code 23. These color markers, geometrical markers, and QR code 23 may be similar to those described in the example of **Fig. 1****.**

In the example of fig. 2, when the detachable portion 4a is removed, the remaining part 2b of the calibration reference 2 defines a boundary around an opening through which a part 3a of the first layer 3 is exposed. If the first layer 3 is transparent, this enables direct observation of the skin portion underneath the dressing 1. In this case, images of a skin alteration (such as a wound, ulcer or burn) can be captured without removing the dressing 1, while the calibration reference 2 surrounding the skin alteration ensure that captured images can be geometrically calibrated and color-calibrated.

**Fig. 3** is a schematic top view of a multi-layered dressing 1 comprising a first layer 3 and a detachable second layer 4. The first layer 3 has two sides. A first side of the first layer 3 is configured to interface with the skin, and a second side of the first layer 3 is configured to interface with the second layer 4. In this case, the entire second layer 4 is detachable. Once the second layer 4 is removed, the side of the first layer 3 that is configured to interface with the second layer 4 is fully exposed.

The left part of **fig. 3** shows the rest of the multi-layered dressing 1 after detachment (e.g., by peeling) of the second layer 4. On the right side of the figure, the detached second layer 4 is shown.

This second layer 4 comprises the calibration reference 2. This calibration reference 2 comprises color markers, two-dimensional markers and a QR code 23, which may be similar to those described with reference to figures 1 and 2.

In the example of figure 3, the second layer 4 comprising the calibration reference 2 can be detached either before or after applying the dressing 1 to the skin. However, the second layer 4 may be provided on the side of the first layer 3 that is configured to interface with the skin during use. In this case, the detachable second layer 4 is removed before applying the dressing 1 to the skin.

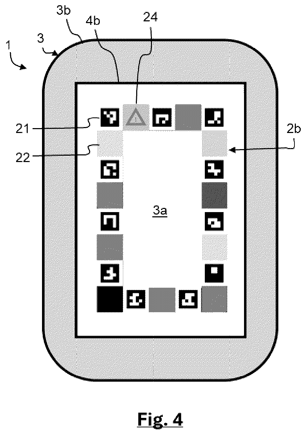

**Fig. 4** is a schematic top view of a multi-layered dressing 1 comprising a first layer 3 and a detachable second layer 4. In this case, a portion 4a of the second layer 4 is detachable (similarly to the example of fig. 2). The detached portion of second layer 4 is not shown.

In this example, the second part 2b of the calibration reference 2 remaining on the dressing 1 after detachment comprises an environment-reactive marker 24. The environment-reactive marker 24 is configured to change in visual appearance in response to a change of an environmental parameter (e.g., pH, temperature, moisture, oxygen levels).

The change in visual appearance may be for instance a color shift, a color darkening, a change in texture, a swelling, a change in transparency.

In this example, the change in visual appearance is a visual cue, specifically a warning symbol, that becomes visible when the environmental parameter exceeds a predefined threshold value.

However, the change in visual appearance may also be gradual or incremental in response to a change of the environmental parameter (e.g., a gradual darkening of color or color shift).

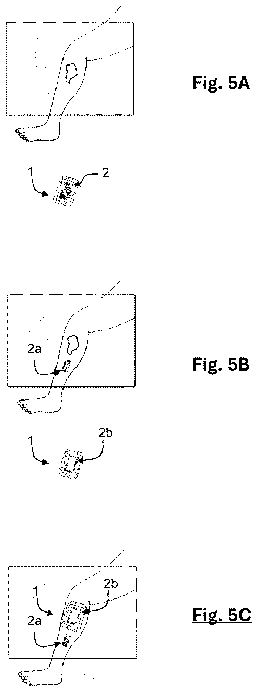

**Figs. 5A, 5B and 5C** illustrate a portion of a subject's skin, which in this case is a lower limb comprising a wound, and a dressing 1 comprising a partially detachable calibration reference 2. This calibration reference 2 may be integrated into a partially detachable second layer 4 similarly to the exemplary dressing 1 of fig. 2. This calibration reference 2 may comprise one or more elements such as geometric markers, color markers, identification markers, and/or environment-reactive markers according to any one of the embodiments herein described. For the sake of clarity, the reference numerals for the dressing layers and for the markers of the calibration reference 2 are not repeated in these figures.

**Fig. 5A** schematically illustrates the subject's lower limb before the multi-layered dressing 1 is adhered on the skin. The dressing 1, which is shown apart from the leg, comprises the calibration reference 2 integrated into a partially detachable second layer 4. The rectangles in figs. 5A, 5B and 5C schematically represents the field of view of an imaging sensor. The portion of the lower limb of the subject intended to be imaged is inside the rectangle.

**Fig. 5B** illustrates the lower limb of fig. 5A in a subsequent step, in which part 2a of the calibration reference 2 has been removed from the dressing and adhered to the skin of the subject near the wound. This part 2a of the calibration reference allows to calibrate images of the skin of the subject using a computer-implemented method.

The dressing 1 is not in contact with the skin and comprises the remaining part 2b of the calibration reference 2.

**Fig. 5C** illustrates the lower limb of figs. 5A and 5B in a further subsequent step, in which the dressing 1 comprising the remaining part 2b of the calibration reference 2 is applied on the wound.

In this case, dual calibration of images can be performed. This dual calibration enables accurate image analysis by providing a calibration reference part 2b on the skin feature for which monitoring is intended (in this example, the wound) and a calibration reference part 2a serving as a control unaffected by wound processes occurring beneath the dressing.

It should be understood that the present invention is not limited to the described embodiments and that variations can be applied without going outside of the scope of the claims.

## Claims

1. A multi-layered dressing (1) configured for monitoring skin changes over time of a skin of a subject by use of a computer-implemented method, the dressing comprising a first layer (3) configured to be in contact with the skin during use of the dressing (1), and a second layer (4) arranged on top of the first layer (3), wherein the second layer (4) comprises a calibration reference (2) configured to be detectable by an imaging sensor when operatively connectable with a computing system for capturing data for use in the computer-implemented method.

2. The multi-layered dressing according to claim 1, wherein
∘ at least one portion (4a) of the second layer (4) is detachably attached to the first layer (3), the detachable portion (4a) of the second layer (4) comprising at least a part (2a) of the calibration reference (2); and/or
∘ the first layer (3) is transparent.

3. The multi-layered dressing according to claim 1 or claim 2, wherein the calibration reference (2) comprises a geometric reference (21).

4. The multi-layered dressing according to claim 3, wherein the geometric reference (21) comprises a one-dimensional marker.

5. The multi-layered dressing according to claim 3, wherein the geometric reference (21) comprises a two-dimensional marker.

6. The multi-layered dressing according to claim 5, wherein the two-dimensional marker comprises one or more of the group of an Aruco marker, a two-dimensional barcode, a QR code.

7. The multi-layered dressing according to claim 3, wherein the geometric reference (21) comprises a three-dimensional marker.

8. The multi-layered dressing according to any of the preceding claims, wherein the calibration reference (2) comprises a color reference (22).

9. The multi-layered dressing according to claim 8, wherein the color reference (22) comprises one or more of the group of a red marker, a yellow marker, a black marker, a brown marker, a grey marker.

10. The multi-layered dressing according to any of the preceding claims, wherein the calibration reference (2) comprises at least one color reference (22) and at least one geometric reference (21) arranged in a grid pattern.

11. The multi-layered dressing according to any of the preceding claims, wherein the calibration reference (2) comprises at least one environment-reactive marker (24) configured to change in visual appearance in response to a change of an environmental parameter.

12. The multi-layered dressing according to claim 11, wherein the change in visual appearance is triggered when the environmental parameter is above a predefined threshold value.

13. The multi-layered dressing according to claim 11 or claim 12, wherein
∘ the environmental parameter comprises one or more of a group of pH, temperature, moisture, oxygen levels; and/or
∘ the change in visual appearance comprises one or more of a group of color shift, change in texture, swelling, change in transparency.

14. The multi-layered dressing according to any one of the preceding claims, wherein the first layer comprises the second layer or the second layer comprises the first layer.

15. Method for manufacturing a multi-layered dressing according to any of the preceding claims, the method comprising the steps of printing the calibration reference (2) on the second layer (4) or on the first layer (3).
